# EUROPEAN PATENT APPLICATION

(11) **EP 1 366 707 A1**
(43) Date of publication of application: **03.12.2003**
(21) Application number: 02788595.3
(22) Date of filing: 01.11.2002
(51) Int. Cl.: A61B 5/02, A61B 5/107, A61B 5/145

(54) **INSTRUMENT FOR MEASURING BIOLOGICAL INFORMATION AND METHOD FOR MEASURING BIOLOGICAL INFORMATION**

(30) Priority: 06.11.2001 JP 2001340211
(71) Applicant: MATSUSHITA ELECTRIC INDUSTRIAL CO., LTD., Kadoma-shi, Osaka 571-8501 (JP)
(72) Inventor: KONDOH, Kazuya, Katano-shi, Osaka 576-0022 (JP); UCHIDA, Shinji, Neyagawa-shi, Osaka 572-0807 (JP)
(74) Representative: Grünecker, Kinkeldey, Stockmair & Schwanhäusser Anwaltssozietät
(86) International application number: JP0211414
(87) International publication number: WO03039363

(57) **Abstract**

Because of using a plurality of light sources and photodetectors, fluctuation of output characteristic among individual light sources and fluctuation of output characteristic among individual photodetectors impede a measurement in a satisfactory precision.

The organism information measuring apparatus comprises an LED (105); a detecting region changer (108) for changing a position of a region of light emitted from the LED (105) that has passed through the organism and has again been emitted toward outside through the organism surface; a photodiode (104) for detecting the light that has passed through the detecting region changer (108); a computing unit (104) for acquiring light information related to the light detected by the photodiode (104) , and generating information related to the organism referring to the light information and sample information; and a data storing unit (101) for storing the sample information.

## Description

### TECHNICAL FIELD

The present invention relates to an organism information measuring apparatus and organism information measuring method, for measuring organism information of a subject such as subcutaneous fat thickness, body fat percentage etc.

### BACKGROUND ART

Up to now various apparatus of noninvasive measurement of subcutaneous fat thickness of a subject have been proposed.

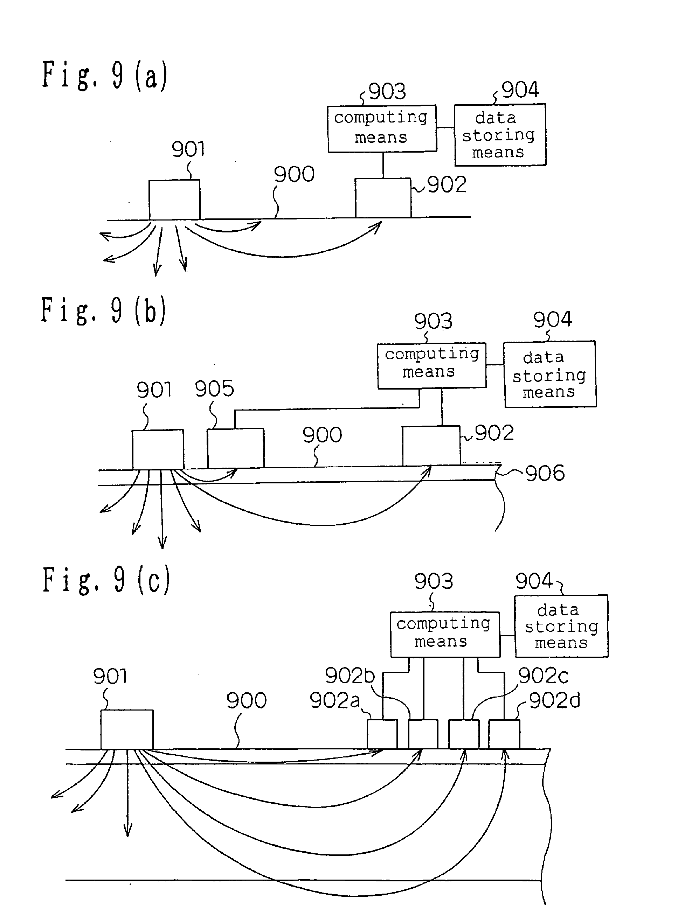

Here, operation of a first working example of an organism information measuring apparatus used for noninvasive measurement of subcutaneous fat thickness (hereinafter referred to as "subcutaneous fat thickness measuring apparatus") according to a conventional technique is schematically shown in Fig. 9(a).

As shown in Fig. 9(a), the subcutaneous fat thickness measuring apparatus according to the first working example is provided with a single light source 901 and a photodetector 902 located on an organism skin 900, computing means 903 connected with the photodetector 902 and data storing means 904 to be utilized by the computing means 903.

In this subcutaneous fat thickness measuring apparatus, the photodetector 902 receives light output from the light source 901 that has passed through inside the organism and reappeared on its surface and converts the light into an electric signal, and then the computing means 903 refers to data stored in the data storing means 904, thus to obtain a subcutaneous fat thickness corresponding to the electric signal value received from the photodetector 902. Here, data that shows a relation between the subcutaneous fat thickness and the electric signal is prepared through establishing a correspondence between an electric signal value measured by a photodetector of a subcutaneous fat measuring apparatus having an equivalent constitution to this subcutaneous fat thickness measuring apparatus from a plurality of organisms serving as testees whose specific values of subcutaneous fat thickness have already been obtained through measurement by for example an MRI or ultrasonic diagnosis apparatus etc., and the subcutaneous fat thickness of the respective testees.

Meanwhile, the light emitted from the light source passes not only through the fat but also through at least a skin of the organism surface, after which the light enters into the photodetector.

Accordingly, since the light received by the photodetector incurs reflection or attenuation because of the skin, correction has to be executed when obtaining the subcutaneous fat thickness of the organism, by correcting the light amount.

Now, operation of a second working example of a subcutaneous fat thickness measuring apparatus according to a conventional technique (for example, refer to JP-A No.2000-155091) is schematically shown in Fig. 9(b).

As shown in Fig. 9(b), the subcutaneous fat thickness measuring apparatus according to the second working example is provided with an auxiliary photodetector 905 in the proximity of the light source 901, in addition to the constitution of the first working example. In this subcutaneous fat thickness measuring apparatus, the auxiliary photodetector 902 receives only such light that has passed through the organism skin 906 and reappeared out of the light output from the light source 901, and converts such light into an electric signal, in addition to the operation in the first working example. Then the computing means 903 executes correction of a light amount received by the photodetector 902, by referring to data stored in advance in the data storing means 904 and thus obtaining an attenuation due to the skin corresponding to the electric signal received from the auxiliary photodetector 902, so that a more accurate subcutaneous fat thickness of the organism can be obtained. Here, data that shows a known relation between thickness and/or color of a skin and an electric signal value is prepared, as in the first working example, through measuring a plurality of organisms as testees whose measured values of skin thickness and/or color have already been obtained through measurement by an MRI or ultrasonic diagnosis apparatus etc. using a subcutaneous fat measuring apparatus having an equivalent constitution to this subcutaneous fat thickness measuring apparatus, thereby establishing a correspondence between an electric signal value according to the photodetector 902 as well as an electric signal value according to the auxiliary photodetector, and a subcutaneous fat thickness of the respective testees.

Further, operation of a third working example of a subcutaneous fat thickness measuring apparatus according to a conventional technique (for example, refer to JP-A No. 11-239573) is schematically shown in Fig. 9(c). The subcutaneous fat thickness measuring apparatus according to the third working example is provided with a single light source 901 and a plurality of photodetectors 902a to 902d located on an organism skin, computing means 903 connected with the photodetectors 902a to 902d and data storing means 904 to be utilized by the computing means 903. In this subcutaneous fat thickness measuring apparatus, the photodetectors 902a to 902d receive light output from the light source 901 that has passed through inside the organism and reappeared on its surface, and convert the light into electric signals, and the computing means 903 refers to data stored in the data storing means 904, thus to obtain a subcutaneous fat thickness corresponding to the electric signal values received from the photodetectors 902a to 902d.

Here, data that shows a known relation between the subcutaneous fat thickness and the electric signal stored in the data storing means 904 is prepared, as in the first and second working examples, through measuring a plurality of organisms as testees whose measured values of subcutaneous fat thickness have already been obtained through measurement by an MRI or ultrasonic diagnosis apparatus etc. using a subcutaneous fat measuring apparatus having an equivalent constitution to this subcutaneous fat thickness measuring apparatus, thereby establishing a correspondence between an electric signal value obtained by the photodetector 902 and a subcutaneous fat thickness of the respective testees.

Also, in the computing means 903, a plurality of subcutaneous fat thickness data respectively corresponding to the photodetectors 902a to 902d is obtained, for which optical paths of the lights respectively received by the photodetectors 902a to 902d are different from one another as shown in Fig. 9(c).

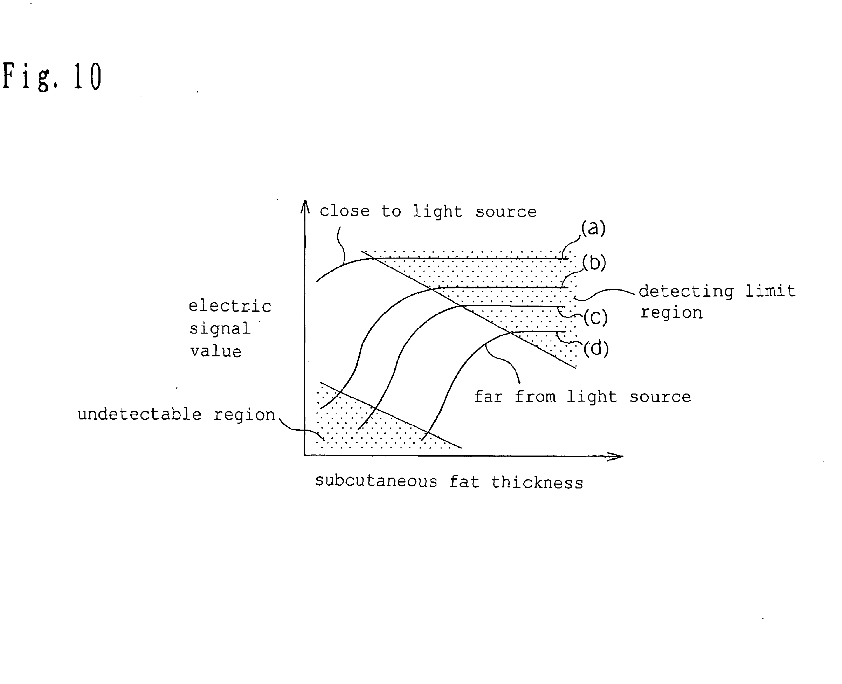

Now Fig. 10 shows curves representing correspondence of subcutaneous fat thickness and electric signal value with respect to each photodetector. In Fig. 10, the curve (a) to curve (d) respectively correspond to electric signal values of the photodetectors 902a to 902d.

As shown by the curve (a) , the electric signal value obtained by the photodetector 902a disposed the closest to the light source 901 rapidly varies within a range where the organism's subcutaneous fat thickness is thin, and quickly reaches the detecting limit (shown by the oblique line portion (detecting limit region) in an upper part of the drawing) at a low value of thickness. On the contrary, as shown by the curve (d) , the electric signal value obtained by the photodetector 902d disposed the farthest from the light source 901 does not arise in a range where the organism's subcutaneous fat thickness is thin and remains in an undetectable state (shown by the oblique line portion (undetectable region) in a lower part of the drawing) , and shows a change only after reaching a certain thickness. This is because the photodetector 902a closer to the light source 901 receives a larger amount of light that has propagated through a shallow portion of the organism, while the photodetector 902d farther from the light source 901 receives more of the light that has propagated through a deep portion of the organism.

In case where the subcutaneous fat is thin, a variation range of the electric signal value output by the photodetector 902a that is close to the light source 901 becomes greater according to a difference of the subcutaneous fat thickness. On the contrary, since the photodetector 902d that is the farthest from the light source 901 receives the light that has propagated through a muscle layer etc. under the subcutaneous fat, the light amount is small and the electric signal value does not make a significant difference. Also, in case where the subcutaneous fat is thick, the electric signal value does not make a significant difference because the photodetector 902a that is close to the light source 901 scarcely detects a difference in a shallow portion of the organism. It is because, on the contrary since a greater difference of light component is produced when propagating through a deep portion of the organism, a difference of the electric signal value output from the photodetector 902d that is the farthest from the light source 901 becomes greater.

In view of the foregoing, it is understood that a correlation exists between a subcutaneous fat thickness and a distance of a photodetector from a light source, and that it is desirable to detect a subcutaneous fat thickness using a photodetector close to the light source in case of an organism having a thin subcutaneous fat thickness, while it is desirable to detect a subcutaneous fat thickness using a photodetector far from the light source in case of an organism having a thick subcutaneous fat thickness.

Accordingly, a distance between a light source and a photodetector has to be an optimum distance in accordance with a subcutaneous fat thickness of an organism. The computing means 903 refers to the curves shown in Fig. 10 to obtain a subcutaneous fat thickness corresponding to electric signal values obtained by the four photodetectors 902a to 902d, wherein among intersection points on a curve that give a correspondence of an electric signal value and a subcutaneous fat thickness those located in the detecting limit region and the undetectable region are excluded and an appropriate photodetector is selected among the photodetectors 902a to 902d, so that a subcutaneous fat thickness of the organism is obtained from the electric signal value of the selected photodetector.

However, these conventional organism information measuring apparatus had the following problems.

Firstly, as described with respect to the second working example, since the auxiliary photodetector is disposed on an organism surface, a detected light has not necessarily propagated through the skin only and a propagating region on the skin detected by the photodetector is different in the strict sense of the word. Therefore, it is impossible to execute an accurate correction of a skin.

Also, in both of the second and third working examples it is necessary to prepare a plurality of photodetectors that are expensive, which makes the organism information measuring apparatus an expensive stuff.

### DISCLOSURE OF THE INVENTION

The present invention has been made in view of the foregoing problems, with an object to provide an accurate organism information measuring apparatus of a low cost that does not require a plurality of neither light sources nor photodetectors.

Also, it is another object of the invention to provide an organism information measuring apparatus and organism information measuring method by which a net reflection rate of a skin is calculated and an accurate correction of the skin can be easily executed.

To achieve the above object, a first invention of the present invention is an organism information measuring apparatus, comprising:
a light source (105) for illuminating an organism surface;
a photoelectric conversion element (104) for receiving light that has passed through a measuring region (109, 203, 303, 405) , out of light emitted from said light source that has propagated through inside said organism and has again been emitted toward outside through said organism surface;
region changing means (106, 108, 201, 301, 404) of changing a position and/or shape of said measuring region;
computing means (102) of acquiring light information related to said light received by said photoelectric conversion element and obtaining information related to said organism through reference to said light information and sample information;
storing means (101) of storing said sample information; wherein
said sample information is defined by sample light information obtained when a sample photoelectric conversion element equivalent to said photoelectric conversion element has received light that has passed through a sample measuring region changed by sample changing region means equivalent to said region changing means out of light emitted from a sample light source, equivalent to said light source toward a testee whose organism-related information is known which has propagated through said testee and has been emitted again toward outside through a surface of said testee, and by said organism-related information of said testee.

A second invention of the present invention is the organism information measuring apparatus as set forth in the first invention of the present invention, wherein said light information includes light amount or luminance distribution.

A third invention of the present invention is the organism information measuring apparatus as set forth in the first or the second invention of the present invention, further comprising light shielding means (110, 204, 302, 404) disposed between said light source and said photoelectric conversion element of preventing direct penetration of light emitted from said light source into said photoelectric conversion element.

A fourth invention of the present invention is the organism information measuring apparatus as set forth in any of the first to the third inventions of the present invention, wherein said region changing means changes a distance between said light source and said measuring region.

A fifth invention of the present invention is the organism information measuring apparatus as set forth in any of the first to the third inventions of the present invention, wherein said region changing means changes a shape of said measuring region and said change of a shape is executed in a separating direction from said light source.

A sixth invention of the present invention is the organism information measuring apparatus as set forth in the fourth invention of the present invention, wherein said region changing means comprises a component (108) made of a flexible material having an opening (109) of a prescribed size disposed in such a manner that a face including said opening makes contact with or comes close to said organism surface; and said measuring region corresponds to said opening.

A seventh invention of the present invention is the organism information measuring apparatus as set forth in the sixth invention of the present invention, wherein said component made of a flexible material further serves as said light shielding means.

An eighth invention of the present invention is the organism information measuring apparatus as set forth in the fourth invention of the present invention, wherein said region changing means comprises two plate-shape components (201a, 201b) provided with a slit (202a, 202b) of a prescribed width, wherein
one and the other of said plate-shape components are disposed in a slidable layer at a position where said slits intersect;
said measuring region corresponds to an opening formed at a position of intersection of said respective slits, and said position is varied according to a sliding motion of said plate-shape components.

A ninth invention of the present invention is the organism information measuring apparatus as set forth in the fifth invention of the present invention, wherein said region changing means comprises a plate-shape components (404) disposed in such a manner that a face thereof makes contact with or comes close to said organism surface; and
an area of said measuring region is varied according to a sliding motion of said plate-shape component.

A tenth invention of the present invention is the organism information measuring apparatus as set forth in the ninth invention of the present invention, wherein said region changing means further comprises a box with an opening including said plate-shape component as one of its faces;
said photoelectric conversion element is disposed inside said box (404, 406) with an opening; and
said light source is disposed outside said box with an opening.

An eleventh invention of the present invention is the organism information measuring apparatus as set forth in any of the first to the tenth inventions of the present invention, wherein said information related to an organism includes subcutaneous fat thickness, and a central wavelength of said light source is in a range of 550nm to 800nm.

A twelfth invention of the present invention is an organism information measuring method, comprising:
an illuminating step of illuminating an organism surface;
a photoelectric conversion step of receiving light that has passed through a measuring region, out of light emitted in said illuminating step that has propagated through said organism and has again been emitted toward outside through said organism surface;
a region changing step of changing a position and/or a shape of said measuring region;
a computing step of acquiring light information related to said light received in said photoelectric conversion step and obtaining information related to said organism through reference to said light information and sample information; and
a storing step of storing said sample information; wherein
said sample information is defined by sample light information obtained in a sample photoelectric conversion step equivalent to said photoelectric conversion step when receiving light that has passed through a sample measuring region changed in a sample region changing step equivalent to said region changing step, out of light emitted from a sample light source equivalent to a light source used in said illuminating step toward a testee whose organism-related information is known which has propagated through said testee has been emitted again toward outside through a surface of said testee, and by said organism-related information of said testee.

A thirteenth invention of the present invention is the organism information measuring method as set forth in the twelfth invention of the present invention, wherein said information related to an organism includes subcutaneous fat thickness, and a central wavelength of said light source is in a range of 550nm to 800nm.

### BRIEF DESCRIPION OF DRAWINGS

Fig. 1(a) is a schematic drawing of an organism information measuring apparatus according to the first embodiment of the present invention;
Fig. 1 (b) is a fragmentary plan view of the organism information measuring apparatus according to the first embodiment of the invention;
Fig. 2(a) is a schematic drawing of an organism information measuring apparatus according to the second embodiment of the invention;
Fig. 2 (b) is a fragmentary plan view of the organism information measuring apparatus according to the second embodiment of the invention;
Fig. 3(a) is a schematic drawing of an organism information measuring apparatus according to the third embodiment of the invention;
Fig. 3 (b) is a fragmentary plan view of the organism information measuring apparatus according to the third embodiment of the invention;
Fig. 3(c) is a schematic drawing for explaining an operation of the organism information measuring apparatus according to the third embodiment of the invention;
Fig. 4(a) is a schematic drawing of an organism information measuring apparatus according to the fourth embodiment of the invention;
Fig. 4 (b) is a fragmentary plan view of the organism information measuring apparatus according to the fourth embodiment of the invention;
Fig. 5 is a schematic drawing of an organism information measuring apparatus according to the fifth embodiment of the invention;
Fig. 6 is a schematic drawing for explaining an operation of the organism information measuring apparatus according to the fifth embodiment of the invention;
Fig. 7 is a schematic drawing for explaining an operation of the organism information measuring apparatus according to the fifth embodiment of the invention;
Fig. 8 is a schematic drawing for explaining an operation of the organism information measuring apparatus according to the fifth embodiment of the invention;
Fig. 9(a) is a schematic diagram showing a first working example of an organism information measuring apparatus according to a conventional technique;
Fig. 9(b) is a schematic diagram showing a second working example of an organism information measuring apparatus according to a conventional technique;
Fig. 9 (c) is a schematic diagram showing a third working example of an organism information measuring apparatus according to a conventional technique;
Fig. 10 is a graph for explaining an operation of an organism information measuring apparatus according to a conventional technique;
Fig. 11 is a graph for explaining an operation of the organism information measuring apparatus according to the third and fourth embodiments of the invention;
Fig. 12 (a) is a schematic drawing for explaining an operation of the organism information measuring apparatus according to the second embodiment of the invention; and
Fig. 12 (b) is a schematic drawing for explaining an operation of the organism information measuring apparatus according to the second embodiment of the invention.

### [Reference Numerals]

- 101: data storing means
- 102: computing means
- 103: display means
- 104: photodiode
- 105: LED (Light Emitting Diode)
- 106: sliding guide unit
- 107: organism surface
- 107a: subcutaneous fat
- 108: detecting region changer
- 109: opening
- 110: extended portion
- 201: detecting region changer
201a plate-shape component
201b plate-shape component
202 slit
202a slit
202b slit
- 203: opening
- 301: detecting region changing means
301a fixed plate
301b fixed plate
301c movable plate
- 302: light shielding means
- 303: detecting region
- 304: skin
- 401: first storing space
- 402: second storing space
- 403: probe container
- 404: shielding plate
- 405: detecting region
- 406: partition wall
- 501: probe container
- 502: shielding plate
- 503: first storing space
- 504: second storing space
- 505: illuminating light switching plate
- 506: measuring region
- 507: partition wall
- 508: window portion
- 901: light source
- 902: photodetector
- 902a: photodetector
- 902b: photodetector
- 902c: photodetector
- 902d: photodetector
- 903: computing means
- 904: data storing means
- 905: auxiliary photodetector

### BEST MODE FOR CARRYING OUT THE INVENTION

Referring to the drawings, the embodiments of the present invention shall be described hereunder.

### Embodiment 1.

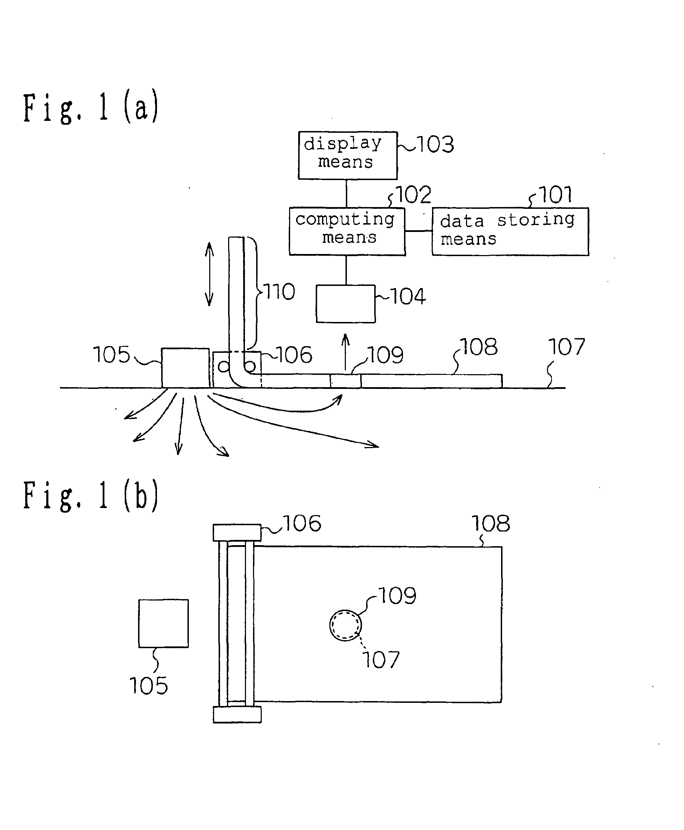

Fig. 1(a) is a schematic drawing of an organism information measuring apparatus according to the first embodiment of the invention. Also, Fig. 1(b) is a fragmentary plan view of the organism information measuring apparatus according to this embodiment. As shown in Fig. 1 (a), the organism information measuring apparatus according to this embodiment is provided with data storing means 101, computing means 102, display means 103, a photodiode 104 connected with the computing means 102, and an LED to be put in close contact with an organism. Here, it is desirable that a central wavelength of the LED 105 is in a range of 550 to 800nm. In this wavelength range subcutaneous fat and a muscle under the subcutaneous fat have different optical propagation characteristics each other and light tends to diffuse more in fat, therefore a difference of light amount received by the photodiode 104 is more apt to be produced by a difference of the fat thickness. Accordingly, the range is appropriate for subcutaneous fat thickness measurement. Also, the organism information measuring apparatus according to this embodiment is provided with a detecting region changer 108 that slides along the organism surface 107 when operated by a user through a sliding guide unit 106. Here, region changing means comprises the sliding guide unit 106 and the detecting region changer 108. The detecting region changer 108 is a plate-shape component made of a flexible material such as rubber, vinyl, bellows, etc., and is provided with a opening 109 as shown in Fig. 1 (a) and (b) . This opening 109 is provided so that the organism surface 107 is exposed therethrough.

Under such constitution, light that has passed through the opening 109, out of light emitted from the LED 105 that has passed through the organism surface 107 has been emitted again toward outside through the organism surface 107, penetrates into the photodiode 104. An amount of the light received by the photodiode 104 is converted into an electric signal value.

The data storing means 101 stores a plurality of data represented by curves showing a relation between subcutaneous fat thickness and electric signal value corresponding to each of a plurality of distances between the LED 105 and the opening 109. This data is represented by a curve formed according to correlation between subcutaneous fat thickness of a plurality of testees accurately measured by an MRI or ultrasonic diagnosis apparatus etc. (organism information of testees) and electric signal values according to light amount obtained through measurement of organism surface of the same testees over their subcutaneous fat thickness by an apparatus equivalent to the organism information measurement apparatus of the invention. The data shown by such curve varies as Fig. 10 showing a conventional example when a distance between the LED 105 and the opening 109 is changed, and in case where this distance is short a thin subcutaneous fat thickness can be measured, while in case where the distance is long a thick subcutaneous fat thickness can be measured. Here, an equivalent apparatus means an apparatus provided with an LED equivalent to the LED 105, a photodiode equivalent to the photodiode 104, and sample region changing means equivalent to the region changing means, according to the embodiment 1 of the invention respectively.

Also, in each of the foregoing constitution, the data storing means 101 is an example of the storing means of the present invention; the computingmeans 102 is an example of the computing means of the present invention; the photodiode 104 is an example of the photoelectric conversion element of the present invention; and the LED 105 is an example of the light source of the present invention. Also, the detecting region changer 108 is an example of the flexible component of the present invention, and its opening 109 is an example of the measuring region of the present invention. Also, the detecting region changer 108 and the sliding guide unit 106 constitute the region changing means of the present invention. Further, the light amount received by the photodiode 104 is an example of the light information of the present invention; the subcutaneous fat thickness is an example of the organism-related information of the present invention; a plurality of data stored in the data storing means, represented by curves showing relation between subcutaneous fat thickness and electric signal value corresponding to each of a plurality of distances between the LED 105 and the opening 109 is an example of the sample information of the present invention. Also, the aforementioned subcutaneous fat thickness of a plurality of testees accurately measured by an MRI or ultrasonic diagnosis apparatus etc. is an example of the organism information related to the testees of the present invention. Also, the light amount obtained through measurement of organism surface of the testees over their subcutaneous fat thickness by an apparatus equivalent to the organism information measurement apparatus of the present invention is an example of the sample light information of the present invention.

Operation of the organism information measuring apparatus having the foregoing constitution according to the embodiment 1 of the present invention shall now be described hereunder and, thereby, an embodiment of the organism information measuring method of the present invention shall be described.

By manipulating the detecting region changer 108 so that a position of the opening 109 is located at a prescribed distance from the LED 105, turning on the LED 105 to illuminate the organism surface 107, and measuring an electric signal value of the photodiode 104 that receives light that has passed through the opening 109, an electric signal value at the prescribed distance can be obtained.

Then, by manipulating the detecting region changer 108 so that the opening 109 comes to a different position than the previous position and turning on the LED 105 as previously done, an electric signal value at a new distance between the LED 105 and the opening 109 is obtained.

Thereafter, by repeating similar operations at different positions of the opening 109, a plurality of electric signal values respectively corresponding to a plurality of distances between the LED 104 and the opening 109 are obtained. The computing means 102 refers to each of the electric signal values obtained as above and the data, thus to obtain a plurality of subcutaneous fat thickness values corresponding to the electric signal values obtained at the respective positions of the opening 109. Among intersection points on the curve that give a correspondence of an electric signal value and a subcutaneous fat thickness, those located in the detecting limit region and the undetectable region are excluded, so that a subcutaneous fat thickness of the organism is obtained from the electric signal value detected when the opening 109 is at an optimum position.

In this way, according to this embodiment, light amount is measured at different distances between the LED 104 and the opening 109 and a plurality of electric signals are obtained based on the respective light amounts, so that an optimum value can be selected to determine a subcutaneous fat thickness of the organism. In other words, a subcutaneous fat thickness measuring range equivalent to that secured in case where a plurality of photoelectric conversion elements are employed can be achieved with a single light source and photoelectric conversion element, by which cost can be lowered.

Also, according to the foregoing description the LED 105 is in contact with the organism surface 107, while the LED 105 may also be disposed with a certain clearance from the organism surface. In this case, it is desirable to utilize an extended portion 110 out of the detecting region changer 108 that is extending upward from the organism surface 107 through the sliding guide unit 106 as a light shielding wall to prevent direct penetration of light from the LED 105 into the photodiode 104. By this arrangement, the extended portion 110 becomes an example of light shielding means of the present invention.

The obtained subcutaneous fat thickness is displayed at a display unit 103. Also, the obtained subcutaneous fat thickness may be transmitted toward outside in a form of digital data, in which case the display unit 103 may be omitted. This equally applies to each of the embodiments to be later described.

Also, it becomes possible to obtain skin thickness etc. in a similar way in addition to subcutaneous fat thickness as organism-related information of the present invention, by selecting an appropriate wavelength of the light source and sample information. This also equally applies to each of the embodiments to be later described.

### Embodiment 2.

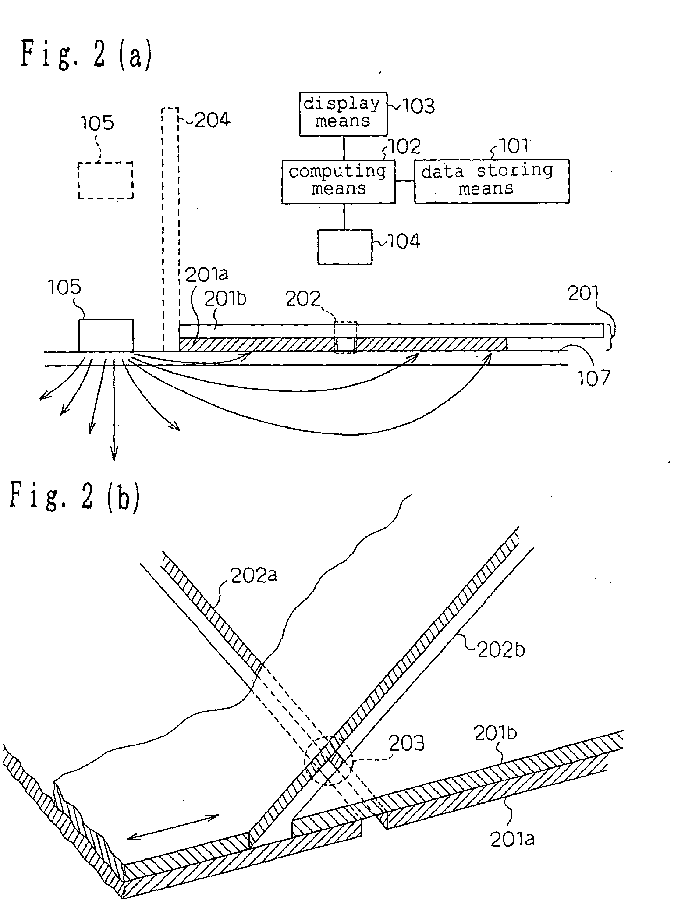

Fig. 2(a) is a schematic drawing of an organism information measuring apparatus according to the embodiment 2 of the present invention. Referring to Fig. 2(a), in the organism information measuring apparatus according to this embodiment, a component identical or corresponding to that of Fig. 1 (a) is denoted by an identical reference numeral, and detailed description thereof shall be omitted. Also, a detecting region changer 201 of this embodiment includes two plate-shape components 201a and 201b. The plate-shape component 201a is in close contact with or extremely close to the organism surface 107, while the plate-shape component 201b is disposed in a form of a slidable layer over the plate-shape component 201a.

Further the plate-shape components 201a and 201b are respectively provided with a slit 202a and 202b at a central portion thereof, so that a opening 203 is formed at an intersection of the slits 202a and 202b when the plate-shape components 201a and 201b are layered. As shown in Fig. 2(b), the organism surface 107 is exposed through the opening 203. Here, under a state in which the detecting region changer 201 is disposed on the organism surface 107 with the plate-shaped component 201a fixed, by holding and manipulating the plate-shape component 201b so that a position of the opening 203 that is an intersection of the slits 202a and 202b is moved to a prescribed distance from the LED 105, turning on the LED 105 to illuminate the organism surface 107, and measuring an electric signal value of the photodiode (photoelectric conversion element) 104 that receives light amount that has passed through the opening 203, an electric signal value at a prescribed distance between the LED 105 and the opening 203 can be obtained.

The data storing means 101 stores a plurality of data represented by curves showing a relation between subcutaneous fat thickness and electric signal value corresponding to each of a plurality of distances between the LED 105 and the opening 203. This data is represented by a curve formed according to correlation between subcutaneous fat thickness of a plurality of testees accurately measured by an MRI or ultrasonic diagnosis apparatus etc. (organism information of testees) and electric signal values according to light amount obtained through measurement of organism surface of the same testees over their subcutaneous fat thickness by an apparatus equivalent to the organism information measurement apparatus of the present invention. The data shown by such curve varies as Fig. 10 showing a conventional example when a distance between the LED 105 and the opening 109 is changed, and in case where this distance is short a thin subcutaneous fat thickness can be measured, while in case where the distance is long a thick subcutaneous fat thickness can be measured. Here, an equivalent apparatus means an apparatus provided with an LED equivalent to the LED 105, a photodiode equivalent to the photodiode 104, and a detecting region changer equivalent to the detecting region changer 201, according to the embodiment 2 of the present invention respectively.

Also, in each of the foregoing constitution, the detecting region changer 201 is an example of the detecting region changer of the present invention, and the plate-shape components 201a and 201b are an example of the two plate-shape components of the present invention. The opening 203 formed at an intersection of the slit 202a of the plate-shape component 201a and the slit 202b of the plate-shape component 201b is an example of the measuring region of the present invention. Meanwhile, since correspondence of other components of identical numerals to those of the embodiment 1 and the respective means of the present invention is similar to the correspondence described in the embodiment 1, description thereof shall be omitted.

Operation of the organism information measuring apparatus having the foregoing constitution according to the embodiment 2 of the present invention shall now be described hereunder and, thereby, an embodiment of the organism information measuring method of the present invention shall be described. However, aspects that are similar to the embodiment 1 shall be omitted, and differences shall mainly be described.

Firstly, under a state in which the detecting region changer 201 is disposed on the organism surface 107 with the plate-shaped component 201a fixed, by holding and manipulating the plate-shape component 201b so that a position of the opening 203 that is an intersection of the slits 202a and 202b is moved to a prescribed distance from the LED 105, turning on the LED 105 to illuminate the organism surface 107, and measuring an electric signal value of the photodiode 104 that receives light amount that has passed through the opening 203, an electric signal value at a prescribed distance between the LED 105 and the opening 203 can be obtained.

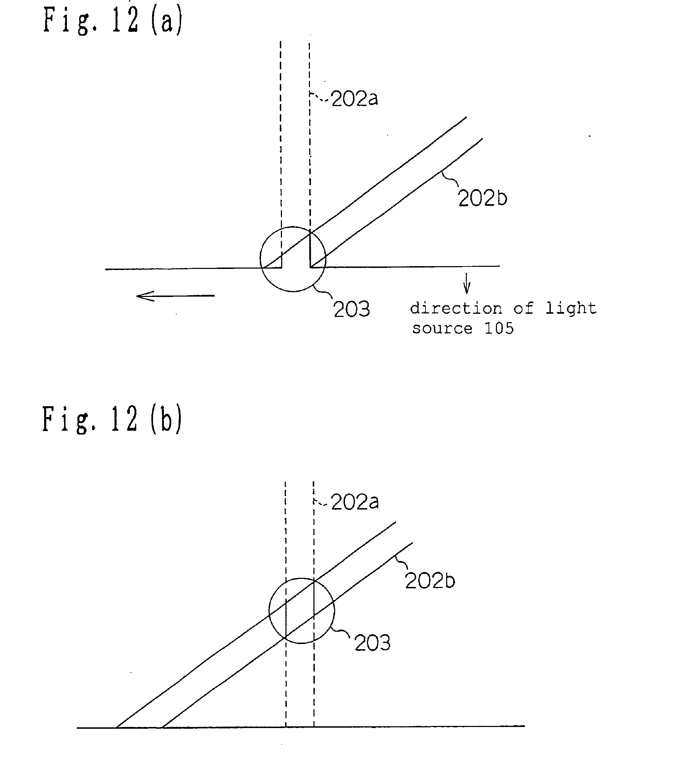

Then, by manipulating the plate-shape component 201b causing it to slide over the plate-shape component 201a so that the intersection position of the slit 202a and the slit 202b is moved to a different position from the previous one. Such status is shown in Figs. 12 (a) and (b) . In Fig. 12(a) the intersection is located at a closest position to the light source 105, but when the plate-shape component 201b is slided in a direction of the arrow in the drawing the opening 203 that is positioned by the intersection point of the slit 202a and the slit 202b moves to a more distant position from the LED 105.

Under such state the LED 105 is turned on to obtain an electric signal value at a new distance between the LED 105 and the opening 203.

Thereafter, by repeating similar operations at different positions of the opening 109, a plurality of electric signal values respectively corresponding to a plurality of distances between the LED 105 and the opening 203 are obtained. The computing means 102 refers to each of the electric signal values obtained as above and the data stored in the data storing means 101, thus to obtain a plurality of subcutaneous fat thickness values corresponding to electric signal values obtained at the respective distances between the LED 105 and the opening (measuring region) 203. As in the embodiment 1, among intersection points on the curve that give a correspondence of an electric signal value and a subcutaneous fat thickness, those located in the detecting limit region and the undetectable region are excluded, so that a subcutaneous fat thickness of the organism is obtained from the electric signal value detected when the opening 203 is at an optimum position.

In this way, according to this embodiment, light amount is measured at different distances between the LED 104 and the opening 203 and a plurality of electric signals are obtained based on the respective light amounts, so that an optimum value can be selected to determine a subcutaneous fat thickness of the organism. In other words, a subcutaneous fat thickness measuring range equivalent to that secured in case where a plurality of photoelectric conversion elements are employed can be achieved with a single light source and photoelectric conversion element, by which cost can be lowered. Also, since the sliding plate-shape component does not directly contact with the organism surface, the opening can be moved without causing any influence to a status of the organism surface, thereby resulting in an accurate detection of light amount.

Also, according to the foregoing description the LED 105 is in contact with the organism surface 107, while in case where the LED 105 is disposed with a certain clearance from the organism surface (shown by dotted lines in Fig. 2(a)), it is desirable to additionally provide a light shielding component 204 on the organism surface, to prevent direct penetration of light from the LED 105 into the photodiode 104. By this arrangement, the light shielding component 204 becomes light shielding means of the present invention.

### Embodiment 3.

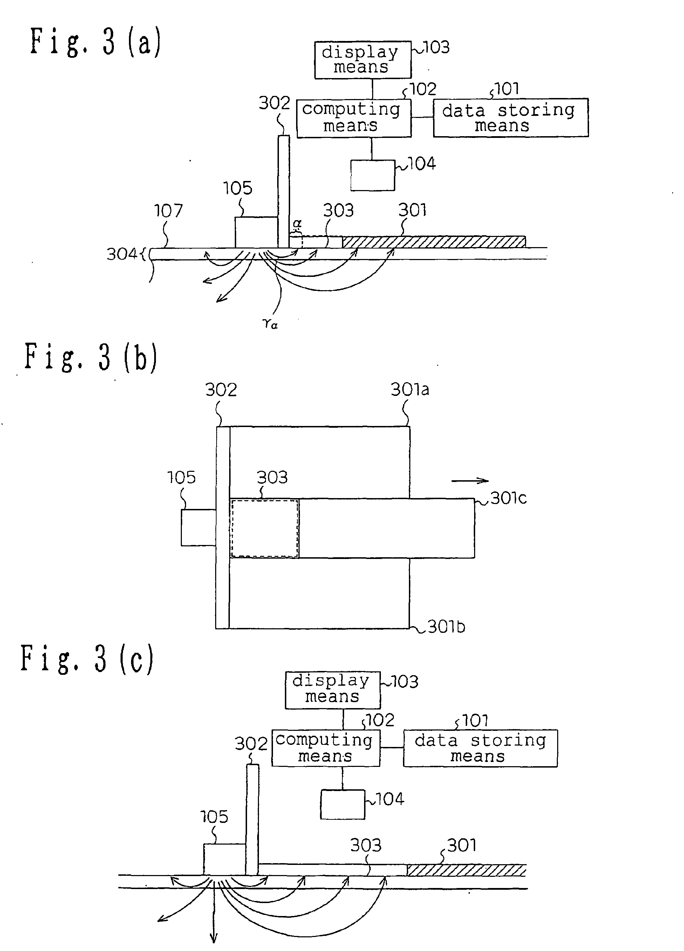

Fig. 3(a) is a schematic drawing of an organism information measuring apparatus according to the third embodiment of the present invention, and Fig. 3(b) is a fragmentary plan view thereof. Referring to Figs. 3(a) and (b), in the organism information measuring apparatus according to this embodiment, a component identical or corresponding to that of Fig. 1 (a) is denoted by an identical reference numeral, and detailed description thereof shall be omitted. Also, the organism information measuring apparatus according to this embodiment is provided with detecting region changing means 301 and light shielding means 302. The detecting region changing means 301 is disposed in close contact with an organism, and through manipulation by a user dimensions of a detecting region 303 over the organism surface 107 can be adjusted. The light shielding means 302 is disposed between the LED 105 and the photodiode 104, thereby preventing direct penetration of light of the LED 105 into the photodiode 104.

The detecting region changing means 301 is provided with a plurality of plate-shape components in combination. The constitution includes two fixed plates 301a and 301b placed with a prescribed clearance therebetween, and a slidable movable plate 301c inserted between the fixed plates 301a and 301b, so that as shown in Fig. 3(b) when the movable plate 301c is moved a detecting region 303 surrounded by the light shielding means 302, fixed plates 301a and 301b and the movable plate 301c is formed, and the organism surface 107 is exposed through the detecting region 303, size of which varies according to a sliding motion of the movable plate 301c.

Also, in each of the foregoing constitution, the detecting region changing means 301 is an example of the detecting region changing means of the present invention, and the movable plate 301c is an example of the plate-shape components of the present invention. Also the detecting region 303 is an example of the measuring region of the present invention and a variation of dimensions of the detecting region 303 is an example of the area of the measuring region of the present invention. Meanwhile, since correspondence of other components of identical numerals to those of the embodiment 1 and the respective means of the present invention is similar to the correspondence described in the embodiment 1, description thereof shall be omitted.

Operation of the organism information measuring apparatus having the foregoing constitution according to the embodiment 3 of the present invention shall now be described hereunder and, thereby, an embodiment of the organism information measuring method of the present invention shall be described. However, aspects that are similar to the embodiment 1 shall be omitted, anddifferences shall mainly be described.

By manipulating the movable plate 301c so as to locate it at a position where the detecting region 303 becomes of a little size, turning on the LED 105 to illuminate the organism surface 107, and measuring an electric signal value of the photodiode 104 that receives light that is an integration of luminance distribution in the detecting region 303, an electric signal value of the photodiode 104 corresponding to a prescribed size of the detecting region 303 can be obtained.

Then, by manipulating the movable plate 301c so as to move it in a direction of separating from the LED 105 thereby making the detecting region 303 larger and turning on the LED 105, an electric signal value of the photodiode 104 corresponding to a larger area of the detecting region 303 is obtained.

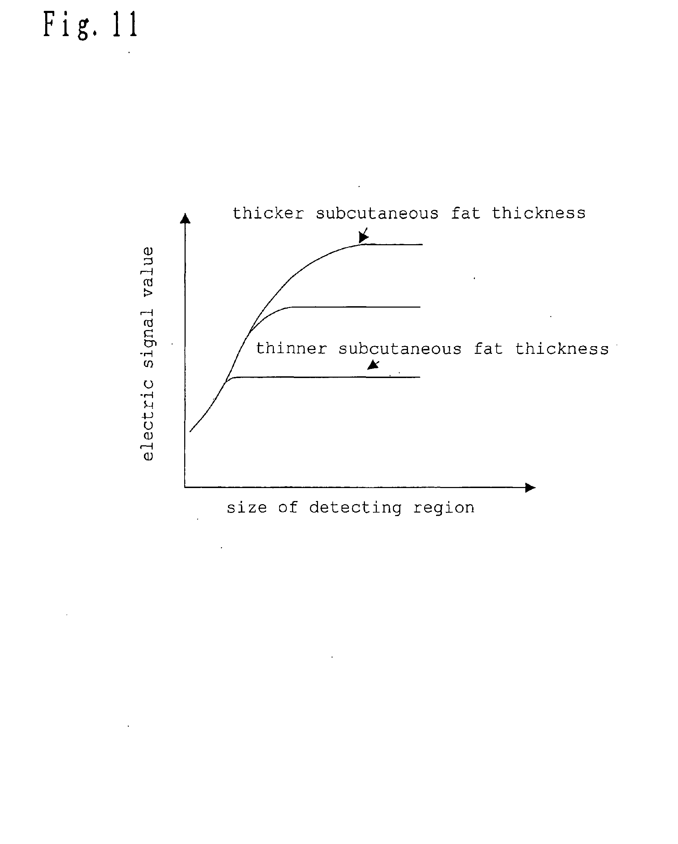

Thereafter, as a result of repeating similar operations at different sizes of the detecting region 303, different electric signal values respectively corresponding to each size of the detecting region 303 can be obtained since the larger the detecting region 303 becomes the more it is integrated as shown in Fig. 3(c). However, increase of the light amount is weakened and the light amount remains close to a constant value after a certain point, since the light attenuates as it goes farther from the LED 105. Such status is shown in the graph of Fig. 11. In Fig. 11, the larger the detecting region 303 becomes the greater the electric signal value becomes. However a curve corresponding to a case where the subcutaneous fat is thin shows that the electric signal value quickly becomes saturated when the detecting region 303 is still small, because a muscle etc. under the subcutaneous fat absorbs a substantial portion of the light amount. On the other hand, since less portion of the light amount is absorbed, a curve corresponding to a case where the subcutaneous fat is thick shows that the electric signal value becomes saturated at a larger size of the detecting region 303 than when the subcutaneous fat is thin.

Accordingly, by gradually changing the size of the detecting region 303 during measurement until the electric signal value becomes saturated, an electric signal value and a size of the detecting region 303 at a point of saturation can be obtained. Then upon finding a subcutaneous fat thickness corresponding to this electric signal value and the size of the detecting region 303 from the data stored in the data storing means 101, subcutaneous fat thickness of each of the subjects can be calculated.

Here, the data stored in the data storing means 101 is obtained from correlation between subcutaneous fat thickness of a plurality of testees accurately measured by an MRI or ultrasonic diagnosis apparatus etc. and an electric signal value and size of the detecting amount region at a saturation point of light amount obtained through measurement of organism surface of the same testees over their subcutaneous fat thickness by an apparatus equivalent to the organism information measurement apparatus of the present invention. This equivalent apparatus means an apparatus provided with an LED equivalent to the LED 105, a photodiode equivalent to the photodiode 104, and sample region changing means equivalent to region changing means 301, according to the embodiment 3 of the present invention respectively.

Further, according to this embodiment, as shown in Fig. 3(a), light amount of light that has passed exclusively through a skin 304 (light path γ α in the drawing) of an organism can be measured when a moving distance of the movable plate 301c is set at a minute value α. Assuming that light amount emitted from the LED (light source) 105 is constant, light transmittance of the skin can be obtained based on light amount of the light that has passed exclusively through the organism skin (light path γ α in the drawing). Accordingly, error due to color of the skin or fluctuation of blood flow can be reduced by utilizing a value obtained by multiplying the electric signal value by the light transmittance of the skin as a real value.

Also, according to the foregoing description the LED 105 is disposed in the proximity of the organism, while the LED 105 may also be disposed in close contact with the organism surface. In this case, the light shielding means 302 may be omitted.

Also, the detecting region changing means 301 may be constituted solely of the movable plate 301c, omitting the fixed plates 301a and 301b.

### Embodiment 4.

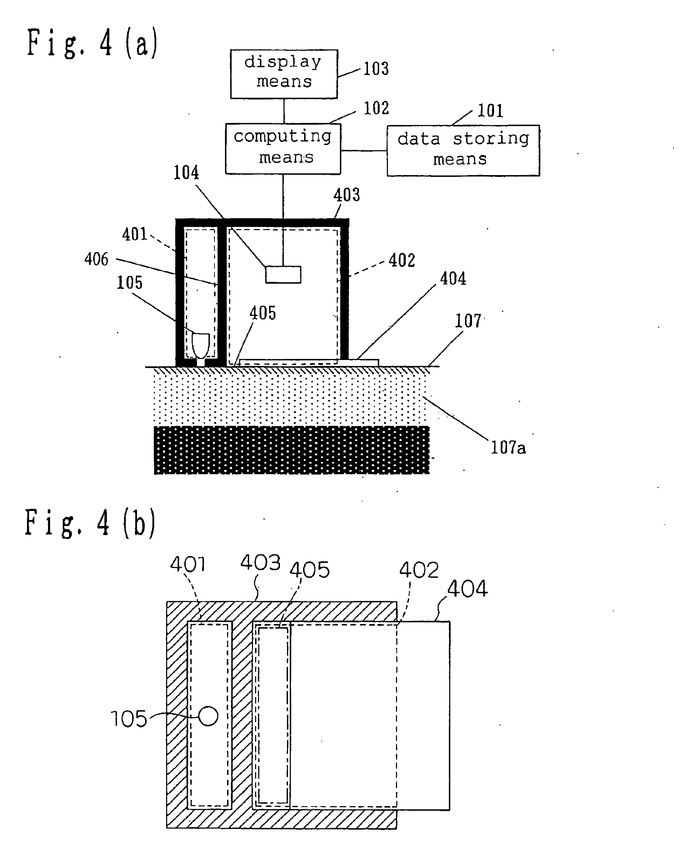

Fig. 4(a) is a schematic drawing of an organism information measuring apparatus according to the fourth embodiment of the present invention, and Fig. 4(b) is a fragmentary plan view thereof. In the organism information measuring apparatus according to this embodiment, a component identical or corresponding to that of Fig. 1 (a) is denoted by an identical reference numeral, and detailed description thereof shall be omitted. Also, in this embodiment the organism information measuring apparatus is provided with a probe container 403 including a first storing space 401 and a second storing space 402 separated by a partition wall 406 from the first storing space 401.

The LED 105 is disposed in the first storing space 401 in the probe container 403. Light emitted from the LED 105 passes through an organism surface 107, and after dispersion and attenuation through skin, subcutaneous fat 107a and a muscle, the light reappears on the organism surface 107. A window is provided at a front portion of the LED 105 for restricting an illuminating area on the organism.

Also, the photodiode 104 is disposed in the second storing space 402 in the probe container 403. Further, a slidable shielding plate 404 is provided at a portion of the second storing space 402 that contacts with the organism surface 107, and side end portions of the shielding plate 404 are in contact with wall faces of the probe conta'iner 403 that form the second storing space 402 as shown in Fig. 4(b), therefore when the front end portion of the shielding plate 404 makes contact with a wall face of the probe container, the organism surface exposed in the second storing space 402 can be fully covered. By moving the shielding plate 404 from the front end portion toward the rear end portion (in a direction of the arrow in the drawing) a detecting region 405 is formed, and an area of the detecting region 405 can be varied to increase in a separating direction from the LED 105. Here, as a result of disposing the photodiode 104 in the second storing space 402 exterior irregular light can be prevented from penetrating into the photodiode, thus enabling high-precision measurement.

Also, in each of the foregoing constitution, those walls in the probe container 403 forming the second storing space 402, including the partition wall 406 and the shielding plate 404, constitute a box with an opening of the present invention. Also the shielding plate 404 is an example of the plate-shape components of the present invention. Also the detecting region 405 is an example of the measuring region of the present invention, and a variation of dimensions of the detecting region 405 is an example of the area of the measuring region of the present invention. Meanwhile, since correspondence of other components of identical numerals to those of the embodiment 1 and the respective means of the present invention is similar to the correspondence described in the embodiment 1, description thereof shall be omitted.

Operation of the organism information measuring apparatus having the foregoing constitution according to the embodiment 4 of the present invention shall now be described hereunder and, thereby, an embodiment of the organism information measuring method of the present invention shall be described. However, aspects that are similar to the embodiment 1 shall be omitted, and differences shall mainly be described.

After manipulating the shielding plate 404 so as to locate it at a position where the detecting region 405 becomes of a little size and turning on the LED 105, an electric signal value of the photodiode 104 that receives light that is an integration of luminance distribution in the detecting region 405 is measured.

Then, after manipulating the shielding plate 404 so as to move it in a separating direction from the LED 105 thereby making the detecting region 405 larger and turning on the LED (light source) 105, an electric signal value of the photodiode 104 corresponding to a larger area of the detecting region 405 is obtained.

Thereafter, as a result of repeating similar operations at different sizes of the detecting region 405, the computing means 102 obtains a plurality of electric signal values respectively corresponding to each size of the detecting region 405. Different electric signal values are obtained for each size of the detecting region 405, since the larger the detecting region 405 becomes the more the light path of light that the photodiode 104 receives is integrated at each measurement, as in the embodiment 3. However, increase of the light amount is weakened and the light amount remains close to a constant value after a certain point, since the lightattenuates as itgoes farther from the LED 105. Such status is shown in the graph of Fig. 11. In Fig. 11, as in the embodiment 3, the electric signal value becomes greater as the size of the detecting region 404 is changed. However a curve corresponding to a case where the subcutaneous fat is thin shows that the electric signal value quickly becomes saturated when the detecting region 404 is still small, because a muscle etc. under the subcutaneous fat absorbs a substantial portion of the light amount. On the other hand, since less portion of the light amount is absorbed, a curve corresponding to a case where the subcutaneous fat is thick shows that the electric signal value becomes saturated at a larger value, at a larger size of the detecting region 404 than when the subcutaneous fat is thin.

Accordingly, by gradually changing the size of the detecting region 404 during measurement until the electric signal value becomes saturated, an electric signal value and a size of the detecting region at a point of saturation can be obtained. Then upon finding a subcutaneous fat thickness corresponding to this electric signal value and the size of the detecting region from a table stored in the data storing means 101, subcutaneous fat thickness of each of the subjects can be calculated.

Also, in the foregoing constitution the probe container 403 is described to include the first storing space 401 and the second storing space 402, while the box with an opening according to the present invention has only to include at least a plate-shape component as a wall, in which photoelectric conversion means can be installed. Therefore, the probe container 403 may be constituted to only include the second storing space 401 in such a manner that the LED 105 is disposed outside the probe container 403.

### Embodiment 5.

In order to accurately measure subcutaneous fat thickness of an organism, it is necessary to consider reflection factor of the organism surface. For such purpose, it is necessary to measure amount of incident light reaching the organism surface (hereinafter referred to as incident light amount) and amount of light that reflects on the organism surface (hereinafter referred to as reflecting light amount) . By measuring these two values a reflection factor of the organism surface can be obtained from a relation of the incident light amount and the reflecting light amount. This embodiment shows a system to obtain the reflection factor.

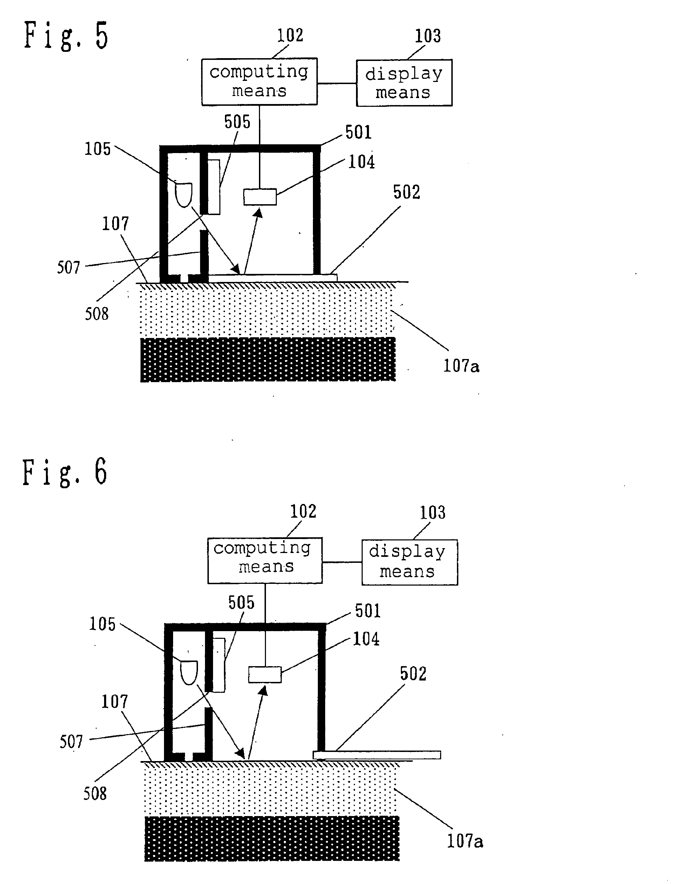

Fig. 5 is a schematic drawing of an organism information measuring apparatus according to the fifth embodiment of the present invention. In the organism information measuring apparatus according to this embodiment, a component identical or corresponding to that of Fig. 1 (a) is denoted by an identical reference numeral, and detailed description thereof shall be omitted. In this embodiment the data storing means 101 is omitted, while the organism information measuring apparatus is provided with a probe container 501 including a first storing space 503 and a second storing space 504 separated from the first storing space 503 by a partition wall 507.

The LED 105 is disposed in the first storing space 503 in the probe container 501. A window is provided at a front portion of the LED 105 for restricting an illuminating area on the organism. Also, a photodiode 104 is disposed in the second storing space 504 in the probe container 501.

Also, in the probe container 501, a main face of a shielding plate 502 opposite to a face that contacts with the organism surface and confronting the photodiode 104 has a known reflection factor K. Here, the shielding plate 502 may be constituted of a material having the reflection factor, or coated with a paint that has the reflection factor, or provided with a film that has the reflection factor. And a portion to be opened or closed by the shielding plate 502 serves as a second lighting window.

Also, the partition wall 507 between the first storing space 503 and the second storing space 504 is provided with a window portion 508 so that the photodiode 104 can measure light of the LED 105 that has reflected on the organism surface, as well as with an illuminating light switching plate 505 for opening and closing the window portion 508.

Further, in each of the foregoing constitutions , those walls in the probe container 501 forming the second storing space 504, including the partition wall 507 and the shielding plate 502, constitute a box with an opening of the present invention and the invention to be later described. Also the shielding plate 502 is an example of the second shielding means of the present invention and the invention to be later described, and the illuminating light switching plate 505 is an example of the first shielding means of the present invention and the invention to be later described. Meanwhile, since correspondence of other components of identical numerals to those of the embodiment 1 and the respective means of the present invention is similar to the correspondence described in the embodiment 1, description thereof shall be omitted.

Operation of the organism information measuring apparatus having the foregoing constitution according to the embodiment 5 of the present invention shall now be described hereunder and, thereby, an embodiment of the organism information measuring method of the present invention shall be described.

Firstly, an electric signal value of the photodiode 104 under a state that the shielding plate 502 that has a known reflection factor K is closed and the illuminating light switching plate 505 is also closed, as shown in Fig. 6, is defined as V0. Under such state, an electric signal value corresponding to a state where exterior light is not penetrating is obtained.

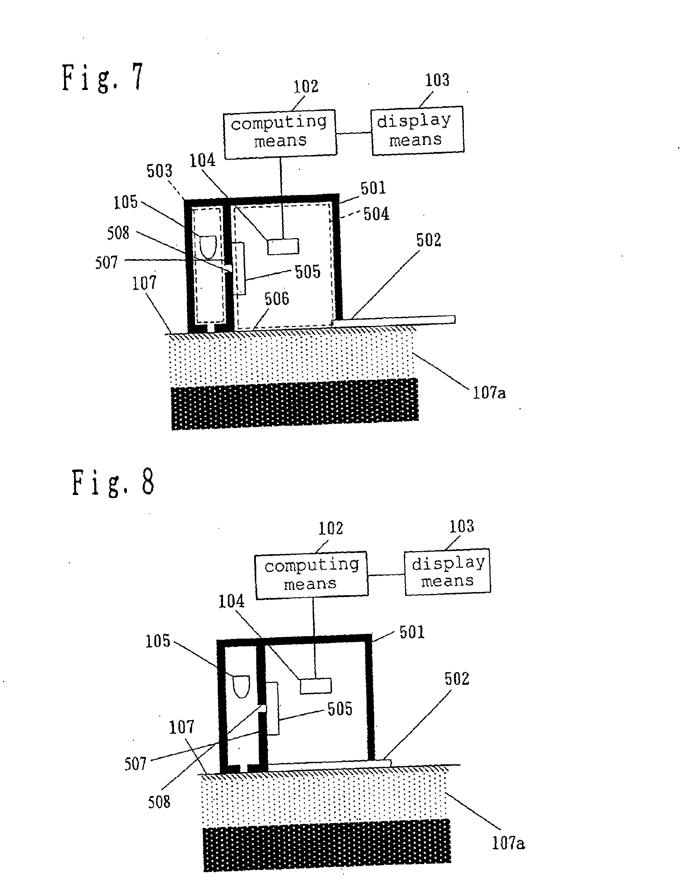

Secondly, as shown in Fig. 7, when the LED 105 is turned on with the illuminating light switching plate 505 open and the shielding plate 502 closed, light from the LED 105 penetrates into the second storing space 503 through the window portion 508 on the partition wall 507 opened by the opening motion of the illuminating light switching plate 505, and is received by the photodiode 104 after reflecting on the shielding plate that has the known reflection factor K. An electric signal value of the photodiode 104 at this moment is defined as Vi. Also, here the window portion 508 corresponds to the first lighting window of the present invention.

Thirdly, as shown in Fig. 8, under a state that both of the illuminating light switching plate 505 and the shielding plate 502 are open, an electric signal value of the photodiode 104, corresponding to a total of a first reflecting light, which is originally emitted from the LED 105 and reflected on the organism surface 107 after penetrating into the second storing space 501 through the window portion opened by the illuminating light switching plate 505 on the partition wall, and propagating light that has appeared on the organism surface 107 through a measuring region 506 created by opening motion of the shielding plate 502 in the second storing space 501 after penetrating through the window and dispersing and attenuating inside the organism, is defined as V1. Also, here the measuring region 506 corresponds to the second lighting window of the present invention.

Fourthly, as shown in Fig. 5, with the illuminating light switching plate 505 closed and the shielding plate 502 opened by a prescribed distance, an output of the photodiode 104, when light of the LED 105 has reached the photodiode 104 as propagating light after dispersing and attenuating inside the organism and appearing through the measuring region 506 created by opening motion of the shielding plate 502 in the second storing space 504, is defined as V2.

Here an electric signal value having information of incident light amount to the organism surface 107 is obtained through a formula of: K × (Vi - V0)

Then, an electric signal value having information of reflecting light amount on the organism surface 107 is obtained through a formula of: V1 - V2

Based on each of the above values, a reflection factor R on the organism surface can be obtained through a formula of: (V1 - V2)/(K × (Vi - V0))

Also, those walls in the probe container 501 forming the second storing space 504, including the partition wall 507 and the shielding plate 502, constitute a box with an opening of the present invention and each of inventions to be later described. Therefore, the portion constituting the first storing space 503 may be omitted from the probe container 501 of the embodiment 5, thereby providing the LED 105 outside the probe container 501.

Now, inventions enforcing the foregoing embodiment 5, invented by the inventor of the present invention shall be described hereunder.

### Invention 1.

An organism information measuring method, employing a light source for illuminating an organism surface; a box with an opening placed outside said light source having a first lighting window capable of directly transmitting light emitted from said light source to be opened or closed by first shielding means and a second lighting window to be opened or closed by second shielding means having a reflecting face of a predetermined reflection factor, for receiving propagating light emitted from said light source that has penetrated into said organism and has reappeared on said another portion of said organism after propagating through therein; a photoelectric conversion element disposed in said box with an opening for detecting light and converting it into an electric signal; and computing means of acquiring said electric signal from said photoelectric conversion element and generating information related to said organism based on said electric signal, comprising:
a first step of detecting by said photoelectric conversion element said propagating light and first reflecting light reflecting from said another portion of said organism, out of said light emitted from said light source with said first shielding means of said box with an opening and said second shielding means opened;
a second step of detecting by said photoelectric conversion element said propagating light, out of said light emitted from said light source with said first shielding means of said box with an opening closed and said second shielding means opened;
a third step of detecting by said photoelectric conversion element second reflecting light reflecting from said reflecting face of said second shielding means, out of said light emitted from said light source with said first shielding means of said box with an opening opened and said second shielding means closed; and
a fourth step of obtaining by said computing means an electric signal value corresponding to an amount of reflecting light reflecting from said organism surface out of said light emitted from said light source, based on an electric signal value obtained by said photoelectric conversion element in said first step and an electric signal value obtained by said photoelectric conversion element in said second step, wherein
a reflection factor of said light emitted from said light source from said organism surface is measured as information related to said organism, by comparison between an electric signal value obtained by said photoelectric conversion element in said fourth step and an electric signal value obtained by said photoelectric conversion element in said third step.

### Invention 2.

The organism information measuring method according to the Invention 1, further comprising:
a fifth step of detecting light and obtaining an electric signal value by said photoelectric conversion element with said first shielding means of said box with an opening and said second shielding means closed, wherein
said reflection factor is corrected by correcting by said computing means an electric signal value obtained by said photoelectric conversion element in said third step and said fourth step using an electric signal value obtained by said photoelectric conversion element in said fifth step.

### Invention 3.

An organism information measuring apparatus comprising: a light source for illuminating an organism surface; a box with an opening placed outside said light source having a first lighting window capable of directly transmitting light emitted from said light source to be opened or closed by first shielding means and a second lighting window to be opened or closed by second shielding means having a reflecting face of a predetermined reflection factor, for receiving propagating light emitted from said light source that has penetrated into said organism and has reappeared on another portion of said organism after propagating through therein; a photoelectric conversion element disposed in said box with an opening for detecting light and converting it into an electric signal; and computing means of acquiring said electric signal from said photoelectric conversion element and generating information related to said organism based on said electric signal, for performing the steps of:
(1) detecting by said photoelectric conversion element said propagating light and first reflecting light reflecting from said another portion of said organism, out of said light emitted from said light source with said first shielding means of said box with an opening and said second shielding means opened;
(2) detecting by said photoelectric conversion element said propagating light, out of said light emitted from said light source with said first shielding means of said box with an opening closed and said second shielding means opened;
(3) detecting by said photoelectric conversion element second reflecting light reflecting from said reflecting face of said second shielding means, out of said light emitted from said light source with said first shielding means of said box with an opening opened and said second shielding means closed;
(4) obtaining by said computing means an electric signal value corresponding to an amount of reflecting light reflecting from said organism surface out of said light emitted from said light source, based on an electric signal value obtained by said photoelectric conversion element in said step (1) and an electric signal value obtained by said photoelectric conversion element in said step (2), wherein
   a reflection factor of said light emitted from said light source from said organism surface is measured as information related to said organism, by comparison between an electric signal value obtained by said photoelectric conversion element in said step (4) and an electric signal value obtained by said photoelectric conversion element in said step (3).

### Invention 4.

The organism information measuring apparatus according to the Invention 3, further comprising:
(5) detecting light and obtaining an electric signal value by said photoelectric conversion element with said first shielding means of said box with an opening and said second shielding means closed, wherein
   said reflection factor is corrected by correcting by said computing means an electric signal value obtained by said photoelectric conversion element in said step (3) and said step (4) using an electric signal value obtained by said photoelectric conversion element in said step (5).

According to the respective inventions, a more precise organism information measuring apparatus and organism information measuring method wherein a reflection factor of the organism surface is taken into account can be accomplished. Here, among the respective components and means in the respective foregoing inventions and the respective components and means of the present invention, those having an identical function are denoted by the identical name.

### Embodiment 6.

The organism information measuring apparatus according to the embodiment 6 is either the organism information measuring apparatus of embodiment 5 with an addition of the data storing means 101, or the organism information measuring apparatus of embodiment 4 wherein the shielding plate 404 is substituted with the shielding plate 502 having a known reflection factor in the embodiment 5. Accordingly, since the embodiment 6 is similar to the embodiments 4 and 5 except contents of the operation of the data storing means 101 and computing operations of the computing means 102, Figs. 4 to 8 shall be used for description thereof, and detailed description shall be omitted.

Operation of the organism information measuring apparatus having the foregoing constitution according to the embodiment 6 of the present invention shall now be described hereunder and, thereby, an embodiment of the organism information measuring method of the present invention shall be described.

After obtaining a reflection factor of the organism surface 107 as described in the embodiment 5, a component that has penetrated into the subcutaneous fat 107a (1-R) × (K × (Vi-V0)) out of an output of the LED 105 is obtained, utilizing the reflection factor.

Then, electric signal values of the photodiode 104 are obtained for different size of the detecting region 404 as in the embodiment 4.

Then an amount of reflecting light is corrected with the skin factor by dividing the electric signal value by the component that penetrated into the subcutaneous fat 107a (1-R) × (K × (Vi - V0)), the quotient of which is the corrected value, and the subcutaneous fat thickness of the organism is obtained by referring to the data stored in the data storing means 101.

Also, the obtained subcutaneous fat and body fat percentage are shown in. the display unit 103. Further, the display unit 203 may be omitted and instead the obtained subcutaneous fat thickness and body fat percentage may be transmitted outside in a form of digital data.

Also, this embodiment 6 may be an example of the following invention achieved by the inventor of the present invention.

### Invention 5.

An organism information measuring apparatus comprising: a light source for illuminating an organism surface; a box with an opening placed outside said light source having a first lighting window capable of directly transmitting light emitted from said light source to be opened or closed by first shielding means and a second lighting window an area of which is changed by second shielding means having a reflecting face of a predetermined reflection factor, for,receiving propagating light emitted from said light source that has penetrated into said organism and has reappeared on another portion of said organism after propagating through therein; a photoelectric conversion element disposed in said box with an opening for detecting light and converting it into an electric signal; means of computing for acquiring said electric signal from said photoelectric conversion element and generating information related to said organism according to light information based on said electric signal; and means of storing for storing said sample information, for performing the steps of:
(1) detecting by said photoelectric conversion element said propagating light and first reflecting light reflecting from said another portion of said organism, out of said light emitted from said light source with said first shielding means of said box with an opening and said second shielding means opened;
(2) detecting by said photoelectric conversion element said propagating light, out of said light emitted from said light source with said first shielding means of said box with an opening closed and said second shielding means opened;
(3) detecting by said photoelectric conversion element second reflecting light reflecting from said reflecting face of said second shielding means, out of said light emitted from said light source with said first shielding means of said box with an opening opened and said second shielding means closed;
(4) obtaining by said computing means an electric signal value corresponding to an amount of reflecting light reflecting from said organism surface out of said light emitted from said light source, based on an electric signal value obtained by said photoelectric conversion element in said step (1) and an electric signal value obtained by said photoelectric conversion element in said step (2), wherein
   a reflection factor of said light emitted from said light source from said organism surface is measured by comparison between an electric signal value obtained by said photoelectric conversion element in said step (4) and an electric signal value obtained by said photoelectric conversion element in said step (3);
   information related to the organism is calculated referring to said light information corrected based on the reflection factor of light emitted from light source at said organism surface and said sample information; and said sample information is defined by sample light information obtained when a sample photoelectric conversion element equivalent to said photoelectric conversion element has received light that has passed through a sample lighting window equivalent to said second lighting window, out of light emitted from a sample light source equivalent to said light source toward a testee whose organism-related information is known.
   Also, in the respective foregoing embodiments, the LED 105 is claimed to be an example of the light source of the present invention and former inventions, while a halogen light or laser may be employed other than an LED, as the light source of the present invention and former inventions. Also, the photodiode 104 is an example of the photoelectric conversion element of the present invention and former inventions, while a CCD or Cds etc. may be employed. Also, the region changing means of the present invention is not limited to those described in the foregoing embodiments, and other devices such as an electric shutter or diaphragm of a mechanical shutter may be employed to change a size or shape of the measuring region. Further, the first shielding means and second shielding means may also be substituted with other constitutions for opening and closing operation, as the region changing means. Furthermore, the electric signal value based on light detected by the photodiode 104 is claimed to be an example of the light information of the present invention and former inventions, while light information of the present invention and former inventions may be light amount, or other data related to luminance distribution obtained based on the light amount.

In addition, in case where height and weight values of testees are obtainable in parallel to the measurement of subcutaneous fat thickness by the organism information measuring apparatus of the present invention and former inventions, body fat percentage can also be statistically calculated.

### INDUSTRIAL APPLICABILITY

As is apparent in view of the foregoing descriptions, according to the present invention an organism information measuring apparatus of a higher precision can be obtained without using a plurality of photodetectors that is an expensive stuff.

## Claims

1. An organism information measuring apparatus, comprising:
a light source for illuminating an organism surface;
a photoelectric conversion element for receiving light that has passed through a measuring region, out of light emitted from said light source that has propagated through inside said organism and has again been emitted toward outside through said organism surface;
region changing means of changing a position and/or shape of said measuring region;
computing means of acquiring light information related to said light received by said photoelectric conversion element and obtaining information related to said organism through reference to said light information and sample information;
storing means of storing said sample information; wherein
said sample information is defined by sample light information obtained when a sample photoelectric conversion element equivalent to said photoelectric conversion element has received light that has passed through a sample measuring region changed by sample changing region means equivalent to said region changing means out of light emitted from a sample light source, equivalent to said light source toward a testee whose organism-related information is known which has propagated through said testee and has been emitted again toward outside through a surface of said testee, and by said organism-related information of said testee.

2. The organism information measuring apparatus as set forth in Claim 1, wherein said light information includes light amount or luminance distribution.

3. The organism information measuring apparatus as set forth in Claim 1 or 2, further comprising light shielding means disposed between said light source and said photoelectric conversion element of preventing direct penetration of light emitted from said light source into said photoelectric conversion element.

4. The organism information measuring apparatus as set forth in any of Claims 1 to 3, wherein said region changing means changes a distance between said light source and said measuring region.

5. The organism information measuring apparatus as set forth in any of Claims 1 to 3, wherein said region changing means changes a shape of said measuring region and said change of a shape is executed in a separating direction from said light source.

6. The organism information measuring apparatus as set forth in Claim 4, wherein said region changing means comprises a component made of a flexible material having an opening of a prescribed size disposed in such a manner that a face including said opening makes contact with or comes close to said organism surface; and said measuring region corresponds to said opening.

7. The organism information measuring apparatus as set forth in Claim 6, wherein said component made of a flexible material further serves as said light shielding means.

8. The organism information measuring apparatus as set forth in Claim 4, wherein said region changing means comprises two plate-shape components provided with a slit of a prescribed width, wherein
one and the other of said plate-shape components are disposed in a slidable layer at a position where said slits intersect;
said measuring region corresponds to an opening formed at a position of intersection of said respective slits, and said position is varied according to a sliding motion of said plate-shape components.

9. The organism information measuring apparatus as set forth in Claim 5, wherein said region changing means comprises a plate-shape components disposed in such a manner that a face thereof makes contact with or comes close to said organism surface; and
an area of said measuring region is varied according to a sliding motion of said plate-shape component.

10. The organism information measuring apparatus as set forth in Claim 9, wherein said region changing means further comprises a box with an opening including said plate-shape component as one of its faces;
said photoelectric conversion element is disposed inside said box with an opening; and
said light source is disposed outside said box with an opening.

11. The organism information measuring apparatus as set forth in any of Claims 1 to 10, wherein said information related to an organism includes subcutaneous fat thickness, and a central wavelength of said light source is in a range of 550nm to 800nm.

12. An organism information measuring method, comprising:
an illuminating step of illuminating an organism surface;
a photoelectric conversion step of receiving light that has passed through a measuring region, out of light emitted in said illuminating step that has propagated through said organism and has again been emitted toward outside through said organism surface;
a region changing step of changing a position and/or a shape of said measuring region;
a computing step of acquiring light information related to said light received in said photoelectric conversion step and obtaining information related to said organism through reference to said light information and sample information; and
a storing step of storing said sample information; wherein
said sample information is defined by sample light information obtained in a sample photoelectric conversion step equivalent to said photoelectric conversion step when receiving light that has passed through a sample measuring region changed in a sample region changing step equivalent to said region changing step, out of light emitted from a sample light source equivalent to a light source used in said illuminating step toward a testee whose organism-related information is known which has propagated through said testee has been emitted again toward outside through a surface of said testee, and by said organism-related information of said testee.

13. The organism information measuring method as set forth in Claim 12, wherein said information related to an organism includes subcutaneous fat thickness, and a central wavelength of said light source is in a range of 550nm to 800nm.
